# EUROPÄISCHE PATENTSCHRIFT

(11) **EP 3 352 711 B1**
(45) Veröffentlichungstag und Bekanntmachung des Hinweises auf die Patenterteilung: **19.06.2024**
(21) Anmeldenummer: 16760104.6
(22) Anmeldetag: 05.09.2016
(51) Int. Cl.: A61F 2/60, A61F 2/64, A61F 2/66, A61F 5/01

(54) **ORTHOPÄDIETECHNISCHE GELENKEINRICHTUNG**
ORTHOPEDIC JOINT SYSTEM
DISPOSITIF D'ARTICULATION POUR L'ORTHOPÉDIE

(30) Priorität: 24.09.2015 DE 102015116149
(43) Veröffentlichungstag der Anmeldung: 01.08.2018
(73) Patentinhaber: Ottobock SE & Co. KGaA, 37115 Duderstadt (DE)
(72) Erfinder: PUSCH, Martin, 37115 Duderstadt (DE); STORCK, Karl-Otto, 37115 Duderstadt (DE)
(74) Vertreter: Gramm, Lins & Partner Patent- und Rechtsanwälte PartGmbB
(86) Internationale Anmeldenummer: PCT/EP2016/070865
(87) Internationale Veröffentlichungsnummer: WO 2017/050552

(56) Entgegenhaltungen:
- EP-A2- 1 072 240
- WO-A1-2015/165981
- WO-A2-01/17466
- US-A1- 2005 015 156

## Beschreibung

Die Erfindung betrifft eine orthopädietechnische Gelenkeinrichtung mit einem Oberteil, an dem obere Anschlussmittel zur Festlegung an einem Patienten angeordnet sind, und einem gelenkig um eine Schwenkachse an dem Oberteil befestigten Unterteil. Die orthopädietechnische Gelenkeinrichtung ist insbesondere als ein künstliches Prothesenkniegelenk oder Orthesenkniegelenk oder Prothesen- oder Orthesenellenbogengelenk ausgebildet, ist jedoch nicht auf diese Ausgestaltungen beschränkt. Bei einer Ausgestaltung als Sperrkniegelenk wird ein Stehen und gegebenenfalls Gehen mit einem verriegelten Gelenk ermöglicht, nach dessen Freigebe ist jedoch eine Verschwenkbarkeit des Oberteils relativ zu dem Unterteil gegeben. Die Gelenkeinrichtung kann jedoch auch als sogenanntes dynamisches Gelenk eingesetzt werden, mit dem spastische Kontrakturen entgegengewirkt wird oder über das mittels Dehnübungen die Kontrakturen therapiert werden können Die WO 2015/165981 A1 offenbart die Merkmale des Oberbegriffs des Anspruchs 1.

Sogenannte Sperrkniegelenke werden häufig bei wenig mobilen Patienten eingesetzt, bei denen eine Einschränkung der Mobilität beispielsweise durch Erkrankungen der unteren Extremitäten begründet sein kann. Aufgrund einer eingeschränkten Mobilität besteht hier ein Bedarf lediglich an Kniegelenken, die ein einschränkungsfreies Sitzen ermöglichen und bei Mobilitätsaktionen ein Größtmaß an Sicherheit bereitstellen. Dazu sehen diese Kniegelenke in der Regel nur zwei Zustände vor, nämlich eine verriegelte Streckstellung und eine Entriegelungsstellung, in der das Kniegelenk frei bewegbar ist. Eine Anpassung der Dämpfung in einer Schwungphase oder Standphase ist nicht vorgesehen. Ein solches Kniegelenk in Gestalt eines Prothesenkniegelenkes ist in der DE 20 2006 007 641 U1 beschrieben. Zur Entriegelung eines solchen Prothesenkniegelenkes ist es notwendig, einen Entriegelungsmechanismus per Hand zu betätigen.

Die DE 10 2008 024 747 A1 betrifft eine orthopädische Einrichtung mit einem Gelenk, das ein Oberteil schwenkbar mit einem Unterteil verbindet. An dem Oberteil sind Anschlussmittel zur Befestigung einer Gliedmaße angeordnet. Eine Verriegelungseinrichtung verhindert eine Beugebewegung des Oberteils relativ zu dem Unterteil, wobei die Verriegelungseinrichtung aktiv durch den Nutzer der orthopädischen Einrichtung betätigbar ist. Eine Steuereinrichtung ist der Verriegelungseinrichtung zugeordnet, die mit zumindest einem Sensor an der orthopädischen Einrichtung verbunden ist, sodass in Abhängigkeit von dem Sensorsignal die Verriegelungseinrichtung automatisch entriegelt oder gesperrt wird.

Die DE 103 51 916 A1 betrifft ein Prothesenkniegelenk mit einem Oberteil und einem schwenkbar daran gelagerten Unterteil und einer dazwischen angeordneten Widerstandseinrichtung, die als Verriegelung gegen eine Flexionsbewegung wirkt und in Abhängigkeit von einem Kniewinkel in einem festgelegten Winkelbereich eine Flexion sperrt und außerhalb des Winkelbereiches eine freie Beweglichkeit ermöglicht.

Die DE 20 2004 008 014 U1 betrifft eine Schwungphasensteuervorrichtung für ein künstliches Kniegelenk mit einer Kolbenzylindereinheit mit zwei Kammern und zwei Drosseln zur Drosselung oder Sperrung eines Durchgangs eines Fluids von der ersten Kammer in die zweite Kammer. Es sind Einrichtungen vorhanden, die die Drosseln bei Erreichen eines bestimmten Druckes in den Kammern schließen.

Die WO 01/17466 A2 betrifft eine Prothese einer unteren Extremität mit einer Hüftbefestigung und Oberschenkel- und Schienbeinkomponenten, die über ein Kniegelenk miteinander verbunden sind, wobei ein Hüftgelenk die Hüftbefestigung mit der Oberschenkelkomponente verbindet. Ein einstellbarer Hüftflexionsantriebsmechanismus ist angeordnet, um ein dynamisch einstellbares Flexionsmoment auf das Hüftgelenk auszuüben.

Die US 2005/0015156 A1 betrifft eine Steuerung eines Prothesenkniegelenkes mit einem Hydraulikdämpfer, der über eine Relativbewegung des Oberschenkelschaftes zu einem Gelenksoberteil geschaltet werden kann. An dem Gelenkoberteil ist die Kolbenstange des Hydraulikdämpfers angeordnet. Zwischen dem Oberteil und dem Oberschenkelschaft sind zwei parallele Laschen angeordnet, über die das Oberteil und der Oberschenkelschaft parallel zueinander verschwenkt werden. Zwischen den beiden Lenkern ist ein Ventil angeordnet, das aufgrund der Längenänderung zwischen den Anlenkpunkten geöffnet oder geschlossen wird.

Aufgabe der vorliegenden Erfindung ist es, eine orthopädietechnische Gelenkeinrichtung, z.B. ein künstliches Kniegelenk, bereitzustellen, die mit einer erhöhten Sicherheit entriegelt werden kann und die es ermöglicht, eine Anpassung der Funktionalität an die wesentlichen Nutzungszustände vorzunehmen.

Erfindungsgemäß wird diese Aufgabe durch eine orthopädietechnische Gelenkeinrichtung mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Die orthopädietechnische Gelenkeinrichtung mit einem Oberteil, an dem obere Anschlussmittel zur Festlegung an einem Patienten angeordnet sind, und einem gelenkig um eine Schwenkachse an dem Oberteil befestigten Unterteil sieht vor, dass zwischen dem Oberteil und dem Unterteil eine flexionsmomentgesteuerte Halteeinrichtung angeordnet ist, die eine Flexion sperrt und bei Überschreiten eines vorbestimmten Flexionsmomentes um die Schwenkachse die Flexion freigibt. Die Halteeinrichtung weist zumindest eine Magnetkopplung und/oder eine Saugnapfkopplung und/oder eine Schnappfeder und/oder eine hydraulische Sperreinheit mit einem schaltbaren Sperrventil auf. Übliche Sperrgelenke sehen eine formschlüssige Verriegelung des Oberteils gegenüber dem Unterteil vor, so dass die Verriegelung zwischen dem Oberteil und dem Unterteil unabhängig von einem aufgebrachten Flexionsmoment beibehalten wird. Erst durch Entriegeln einer formschlüssigen Verriegelungseinrichtung, beispielsweise eines Schiebers, eines Hakens oder eines Stiftes, wird die orthopädietechnische Gelenkeinrichtung, das insbesondere in Gestalt eines Prothesenkniegelenkes oder Orthesenkniegelenkes oder Prothesen- oder Orthesenellenbogengelenkes ausgebildet sein kann, entriegelt und für eine Flexion zugänglich. Grundsätzlich ist es vorgesehen, dass eine freie Flexion nach der Entriegelung, also nach Überwindung der Haltekraft durch Aufbringung eines vordefinierten Flexionsmomentes um die Drehachse der orthopädietechnischen Gelenkeinrichtung, ermöglicht wird, so dass keine Behinderung oder Einschränkung bei der Flexionsbewegung auftritt. Unter freier Flexion wird nicht die vollständig widerstandsfreie Flexion verstanden, da dies bei technischen Systemen nicht möglich ist. Eine freie Flexion liegt auch dann vor, wenn aufgrund von Reibungswiderständen Flexionswiderstände vorliegen. Bei der Ausgestaltung als künstliches Kniegelenk können beim Hinsetzen beide Hände zur Sicherung eingesetzt werden, so dass sich für den Nutzer ein erhöhtes Sicherheitsgefühl einstellt. Wird die orthopädietechnische Gelenkeinrichtung nicht als künstliches Kniegelenk eingesetzt, kann jede Orthese oder Prothese mit einer entsprechenden Halteeinrichtung ausgestattet werden, um eine Flexion bis zu einem vorbestimmten Moment zu sperren und im Rahmen eines Überlastschutzes nach Überschreiten eines Flexionsmomentes das Gelenk freizugeben.

Solange die Halteeinrichtung wirksam ist, beispielsweise durch eine formschlüssige, kraftschlüssige oder andere mechanische Verriegelung, bleibt das Gelenk oder die orthopädietechnische Gelenkeinrichtung gesperrt, das heißt, dass bei üblichen Belastungen, z.B. beim Stehen oder Gehen oder eine Haltestellung eines Ellenbogens, eine Flexion, also eine Verschwenkung des Oberteils relativ zu dem Unterteil um die Schwenkachse, nicht möglich ist. Erst durch Aufbringen einer Kraft auf die Ferse oder das Unterteil allgemein bzw. durch Aufbringen eines Flexionsmomentes, das um die Schwenkachse wirkt, wird die Halteeinrichtung deaktiviert, so dass die Sperre in der künstlichen orthopädietechnischen Gelenkeinrichtung gelöst wird und eine Flexion möglich ist.

Eine Weiterbildung der Erfindung sieht vor, dass zwischen dem Unterteil und dem Oberteil eine Dämpfereinrichtung angeordnet ist, die eine Flexionsbewegung und/oder Extensionsbewegung des Unterteils relativ zu dem Oberteil dämpft. Über die systemimmanente Dämpfung hinaus ist somit eine separate Dämpfereinrichtung vorgesehen, die nach dem Lösen der Sperre eine Dämpfung der Verschwenkbewegung bereitstellt, insbesondere eine Dämpfung in Flexionsrichtung, um so einem Nutzer eine Unterstützung bei der Hinsetzbewegung zu ermöglichen. Bei einer Ausgestaltung als dynamisches Gelenk wird nach Überschreiten eines Flexionsmomentes die Bewegung gedämpft, so dass verhindert wird, dass nach Freigabe der orthopädietechnischen Gelenkeinrichtung eine ungebremste Flexionsbewegung, beispielsweise bei einer Spastik, in die Maximalflexion stattfindet.

Die Dämpfereinrichtung kann zwischen einem hohen Dämpfungswiderstand und einem geringeren Dämpfungswiderstand schaltbar ausgebildet sein, um eine Anpassung an die jeweiligen situativen Notwendigkeiten vornehmen zu können. So kann es wünschenswert sein, eine hohe Dämpfung bereitzustellen, um eine Hinsetzbewegung zu unterstützen oder eine andere Bewegung abzubremsen, während im sitzenden oder flektierten Zustand eine vergleichsweise niedrige Dämpfung eingestellt werden kann, um die während des Sitzens vergleichsweise kleinen Bewegungen im Bein oder Arm ungestört durchführen zu können.

Der Dämpfereinrichtung kann eine axialkraftabhängige Schalteinrichtung zugeordnet sein, die bei Unterschreiten einer auf das Unterteil einwirkenden Axialkraft einen verringerten Dämpfungswiderstand freischaltet. Beim Hinsetzen wirkt auf den Unterschenkel eine vergleichsweise hohe Axialkraft entlang der Längserstreckung des Unterschenkels, beispielsweise einer Unterschenkschiene oder einem Unterschenkelrohr. Eine ähnliche Belastungssituation ergibt sich bei dem Aufstützen der Hand beim Hinsetzen, die Axialkraft wirkt hier in der Unterarmschiene oder im Unterarmrohr. Diese vergleichsweise hohe Axialkraft ist ein Indikator für eine Bewegungssituation, in der ein vergleichsweise hoher Dämpfungswiderstand gewünscht wird. Fällt diese Axialkraft weg, beispielsweise während des Sitzens, bei einem angehobenen Unterschenkel oder einem entlasteten Unterarm, wird ein verringerter Dämpfungswiderstand freigeschaltet und eine freie oder nahezu freie Flexionsbewegung und Extensionsbewegung ermöglicht.

Der Dämpfereinrichtung kann ein mechanischer Schalter oder ein sensorgesteuerter, mit einem Aktuator versehener Schalter zugeordnet sein, so dass die Schaltung entweder rein mechanisch oder elektromechanisch mit einem motorisch betriebenen Aktuator ausgeführt werden kann.

Die Halteeinrichtung kann kraftschlüssig, beispielsweise als Magnet oder Saugnapf ausgebildet sein. Alternativ können formschlüssig wirkende Halteeinrichtungen eingesetzt werden, beispielsweise Klettverschlüsse, Blattfederelemente, die auf einer Rolle entlanggleiten, oder eine Hydraulikeinheit. Bei der Hydraulikeinheit kann bis zu einem vorbestimmten Flexionsmoment ein Überströmen von einer Flexionskammer in eine Extensionskammer gesperrt werden, beispielsweise durch ein vorbelastetes Rückschlagventil, und erst bei Überschreiten eines aufgebrachten Momentes wird der Überströmkanal freigegeben, so dass eine Flexion, gegebenenfalls kombiniert mit einem erhöhten Dämpfungswiderstand, freigegeben wird. Die Vorspannung des Rückschlagventils und damit das Auslösemoment ist vorteilhafterweise einstellbar.

Die gesamte durch die Halteeinrichtung ausgeübte Haltekraft zur Vermeidung einer Flexion der orthopädietechnischen Gelenkeinrichtung ist einstellbar, um eine Anpassung des künstlichen Gelenkes an den jeweiligen Patienten vornehmen zu können.

Eine Weiterbildung der Erfindung sieht vor, dass das Oberteil gegenüber dem Unterteil verschieblich gelagert und erst bei einer bestimmten Position des Oberteils gegenüber dem Unterteil in Axialrichtung eine Deaktivierung der Halteeinrichtung möglich ist. Dazu kann es vorgesehen sein, dass das Oberteil gegenüber dem Unterteil federbelastet ist, also dass zwischen dem Oberteil und dem Unterteil ein Federelement angeordnet ist, dass einer Verschiebung entgegenwirkt und erst bei der Einnahme einer bestimmten Position eine Verschwenkung zulässt.

Zusätzlich zu der Halteeinrichtung kann zwischen dem Oberteil und dem Unterteil ein separater Hydraulikdämpfer angeordnet sein, um nach dem Überwinden des Haltemomentes eine Extensionsbewegung und/oder Flexionsbewegung des Oberteils relativ zu dem Unterteil in einem gewünschten Maße zu dämpfen.

Die Halteeinrichtung kann einen sensorlos arbeitenden Freigabemechanismus aufweisen oder ausbilden, der Teil der Halteeinrichtung ist oder durch die Halteeinrichtung ausgebildet wird. Dadurch ist es möglich, ohne elektrische oder elektronische Komponenten einerseits eine Haltefunktion bereitzustellen, mit der verhindert wird, dass ein Gelenk ungewollt einbeugt, und andererseits ohne Steuerungs- oder Regelungsaufwand, angetriebene Aktuatoren und Sensoren eine Freigabe der Sperrung ab einem vorbestimmten, einstellbaren Flexionsmoment zu ermöglichen. Der Freigabemechanismus kann in der Halteeinrichtung integriert sein und durch eine begrenzte Haltekraft realisiert werden. So kann durch einen magnetischen Halter oder einen Saugnapf gleichzeitig eine Haltefunktion und eine Freigabefunktion durch die begrenzte Haltekraft bereitgestellt werden. Über einen federbelasteten Hebel oder eine Feder kann eine Sperrklinke in der Verriegelungsstellung gehalten werden, bis bei einem vorbestimmten Flexionsmoment die Federbelastung nicht mehr ausreicht, um die Sperrklinke oder eine andere formschlüssig oder kraftschlüssig wirkende Sperreinrichtung in der Verriegelungsstellung zu halten. Dann wird ohne weitere Antriebsleistung oder ohne weiteres Signal die Haltefunktion beendet und das Gelenk freigeben. Sowohl die Halteeinrichtung als auch der Freigabemechanismus arbeiten mechanisch und sind ohne Sensoren oder eine Steuerungseinrichtung funktionsfähig.

Nachfolgend werden Ausführungsbeispiele der Erfindung anhand der beigefügten Figuren erläutert. Es zeigen:
- Fig. 1: eine schematische Darstellung einer orthopädietechnische Gelenkeinrichtung mit einer magnetischen Halteeinrichtung;
- Fig. 2: eine Variante der Figur 1 mit einer unterdruckbasierten Halteeinrichtung;
- Fig. 3: eine Variante der Erfindung mit einer federbelasteten Halteeinrichtung;
- Fig. 4: eine Variante der Erfindung mit einer hydraulischen Halteeinrichtung;
- Fig. 5: einer weitere Variante der Figur 1; sowie
- Fig. 6-8: Varianten der Figuren 1 bis 3 mit einem zusätzlichen Hydraulikdämpfer.

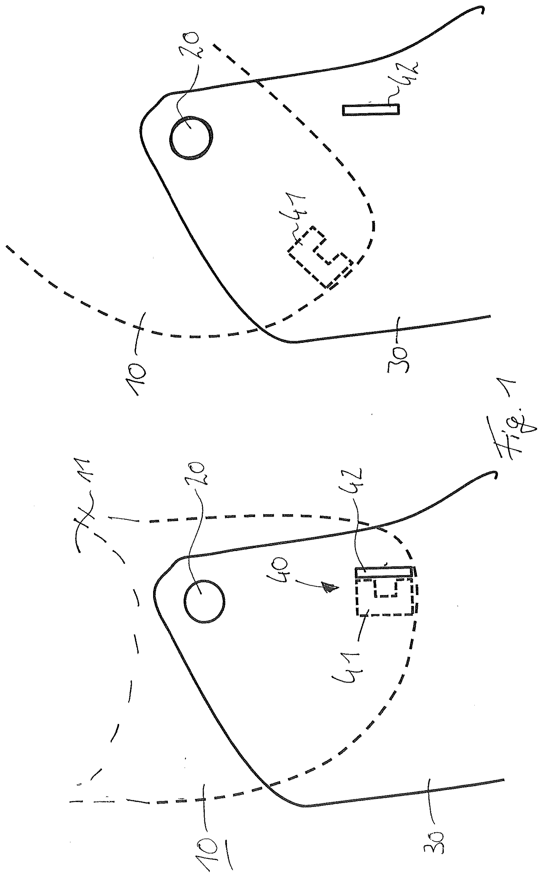

Figur 1 zeigt in einer schematischen Schnittdarstellung eine orthopädietechnische Gelenkeinrichtung in Gestalt eines künstliches Kniegelenkes mit einem Oberteil 10, an dem obere Anschlussmittel 11 in Gestalt eines Oberschenkelschaftes zur Festlegung an einem Patienten angeordnet sind. Alternativ zu einer Ausgestaltung als Prothesenkniegelenk kann das künstliche Kniegelenk auch als ein Orthesenkniegelenk ausgebildet sein, bei dem das Oberteil 10 als eine Oberschenkelschiene und die oberen Anschlussmittel als Manschette, Lasche, Gurt oder dergleichen ausgebildet sind, um die Orthese mit der Oberschenkelschiene an dem Oberschenkel des Patienten anzulegen. Alternativ zu einer Ausgestaltung als künstlichen Kniegelenk sind andere Anwendungen für die orthopädietechnische Gelenkeinrichtung vorgesehen und von der Erfindung umfasst, zum Beispiel für Prothesen und/oder Orthesen für obere und untere Extremitäten wie Ellenbogenorthesen, Handgelenksorthesen sowie Prothesen für Knie, Ellenbogen oder Hände.

An dem Oberteil 10 ist eine Schwenkachse 20 ausgebildet, um die ein an dem Oberteil 10 festgelegtes Unterteil 30 schwenkbar gelagert ist. Das Unterteil 30 kann als Unterschenkelschiene mit Befestigungsmitteln zum Festlegen der Unterschenkelschiene an dem Unterschenkel bei einer Orthesenausgestaltung ausgebildet sein. Die Befestigungsmittel können als Gurte, Manschetten oder dergleichen ausgebildet sein und umschlingen den Unterschenkel, gegebenenfalls kann an der Unterschenkelschiene ein Fußteil angeordnet sein, so dass sich eine KAFO (Knee Ankle Foot Orthosis) ausbildet. Bei einer Ausgestaltung des künstlichen Kniegelenks als Prothesenkniegelenk ist das Unterteil 30 als Unterschenkelrohr ausgebildet, an dem ein nicht dargestellter Prothesenfuß festgelegt ist. Ein korrespondierender Aufbau ist bei Prothesen oder Orthesen der oberen Extremität vorhanden.

Die Schwenkachse 20 ist als durchgehende Schwenkachse ausgebildet, grundsätzlich ist es auch möglich, durch ein Mehrlenkergelenk eine instationäre Gelenkachse auszubilden.

Das Oberteil 10 ragt distal über die Schwenkachse 20 hinaus und weist einen daran befestigten Magneten 41 auf, der an einem Joch 42 anliegt, das an dem Unterteil 30 befestigt ist. Der Magnet 41 und das Joch 42 bilden zusammen die Halteeinrichtung 40, über die das Kniegelenk in der dargestellten gestreckten Position verriegelt wird. In einer solchen verriegelten Stellung ist eine Verschwenkung des Unterteils 30 relativ zu dem Oberteil 10 um die Schwenkachse 20 nicht möglich, der Nutzer oder Patient kann beim Stehen, Gehen oder Sitzen auf ein gesichertes, gestrecktes Kniegelenk vertrauen.

In der rechten Darstellung der Figur 1 ist an das Kniegelenk in einer flektierten, eingebeugten Stellung gezeigt. Durch Aufbringen eines Flexionsmomentes um die Schwenkachse 20, beispielsweise bei einem aufgebrachten Hüftmoment oder einem Fersenauftritt mit ausreichender Belastung eines hinter der Schwenkachse 10 verlaufenden Bodenreaktionskraftvektors, wird die Haltekraft der Halteeinrichtung 40 überschritten, so dass sich der Magnet 41 von dem Joch 42 löst und eine freie Verschwenkbarkeit des Oberteils 10 relativ zu dem Unterteil 30 um die Schwenkachse 20 vorliegt.

In dem dargestellten Ausführungsbeispiel der Figur 1 ist keine weitere Dämpfungskomponente oder Dämpfereinrichtung gezeigt, grundsätzlich ist es auch möglich, dass zwischen dem Oberteil 10 und dem Unterteil 30 eine Dämpfereinrichtung vorgesehen ist und angeordnet ist, über die eine Flexionsbewegung und/oder Extensionsbewegung des Oberteils 10 relativ zu dem Unterteil 30 gedämpft wird. Eine solche Dämpfereinrichtung ist vorteilhafterweise als Hydraulikdämpfer oder Pneumatikdämpfer ausgebildet, grundsätzlich ist es auch möglich, einen Festkörperdämpfer zwischen dem Oberteil 10 und dem Unterteil 30 anzuordnen.

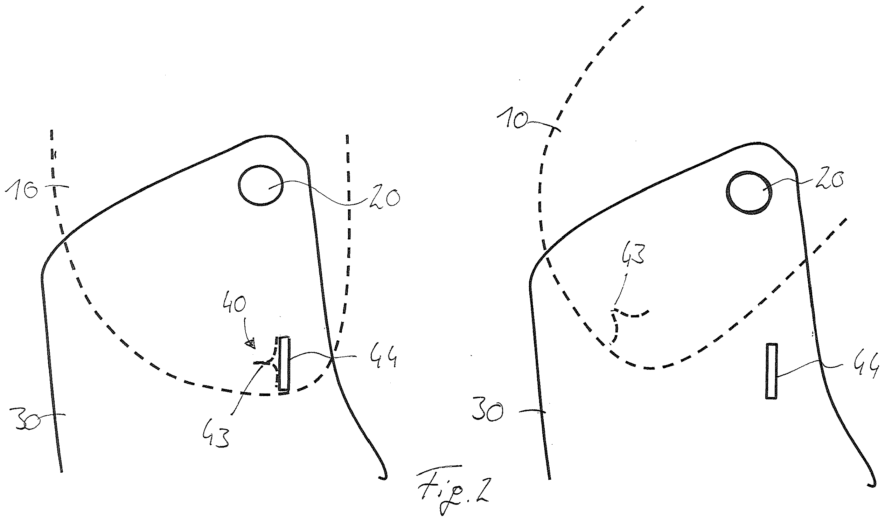

In der Figur 2 ist eine Variante der Figur 1 dargestellt, bei der die Komponenten mit gleichen Bezugszeichen einander entsprechen. Im Unterschied zu der Figur 1 ist in der Figur 2 die Halteeinrichtung 40 vakuumbasiert aufgebaut, die Haltekraft wird über einen Vakuumeffekt verwirklicht. An dem Oberteil 10 ist ein Saugnapf 43 befestigt, der gegen eine glatte Fläche 44, die an dem Unterteil 30 ausgebildet oder angeordnet ist, anliegt und über einen Unterdruck im eingeschlossenen Volumen eine fixierte Anlage bewirkt. Erst bei Überschreiten eines vorgebbaren Flexionsmomentes wird der Saugnapf 43 von der glatten Fläche 44 gelöst, Umgebungsluft tritt in das Volumen zwischen dem Saugnapf 43 und der glatten Fläche 44 ein und führt zu einem Druckausgleich, so dass die Umgebungsluft den Saugnapf 43 nicht mehr an der glatten Fläche 44 gedrückt hält. Die Haltekraft durch den Saugnapf 43 wird überwunden und das Kniegelenk für die Beugebewegung freigegeben.

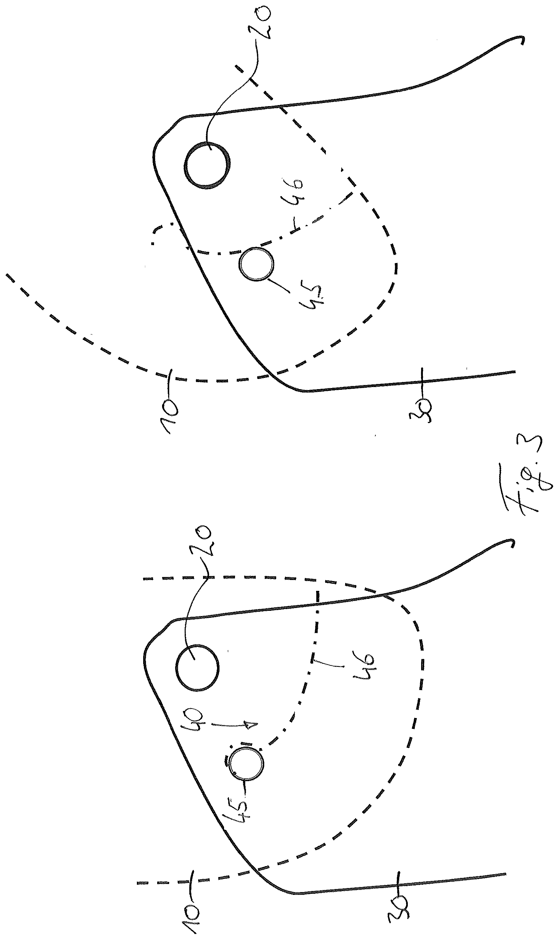

Figur 3 zeigt eine Variante der Erfindung, bei der die Halteeinrichtung 40 federmechanisch ausgebildet ist und aus einer Rolle 45 oder einem Bolzen, der an dem Unterteil 30 befestigt ist, sowie einer Blattfeder 46 besteht, die an dem Oberteil 10 angeordnet ist. Die Blattfeder 46 weist an einem vorderen Ende eine Auswölbung oder Formgebung auf, die korrespondierend zu der Kontur der Rolle 45 ausgebildet ist, so dass im verriegelten Zustand gemäß der linken Darstellung der Figur 3 eine quasiformschlüssige Verriegelung des Kniegelenkes vorliegt. Die Feder 46 drückt aufgrund einer Vorspannung gegen die Rolle 45, so dass das Gelenk, z.B. ein Kniegelenk, Ellenbogengelenk oder Handgelenk in Orthesen- oder Prothesenform, in der Streckstellung gehalten wird, da die Auswölbung oder Krümmung der Feder 46 die Rolle 45 aufnimmt. Bei Aufbringung eines ausreichend hohen Flexionsmomentes um die Schwenkachse 20 wird die Krümmung oder Auswölbung am Ende der Blattfeder 46 aus der Rolle 45 hinausbewegt, die Rolle 45 rollt auf der Feder 46 ab und das Gelenk lässt sich im Wesentlichen frei beugen.

Die dargestellten Ausführungsformen gemäß Figuren 1 bis 3 können auch eine umgekehrte Anordnung der Komponenten der Halteeinrichtung 40 aufweisen. Der Magnet 41 ist dann an dem Unterteil 30 und das Joch 42 an dem Oberteil 10 angeordnet, die glatte Fläche 44 befindet sich an dem Oberteil 10 und der Saugnapf 43 an dem Unterteil 30, ebenso wie die Feder 46 an dem Unterteil 30 und die Rolle 45 an dem Oberteil 10. Ebenso ist es vorgesehen, dass die Haltekraft der Halteeinrichtung 40 jeweils einstellbar ausgebildet ist, bei einer magnetischen Halterung kann dies durch eine Vergrößerung des Abstandes zwischen Joch 42 und Magnet 41, der Einbringung von Material oder aber Verringerung der Kontaktfläche zwischen dem Magneten 41 und dem Joch 42 erfolgen. Eine ähnliche Variation kann bei dem vakuumgestützten Konzept gemäß Figur 2 erfolgen, bei der die Fläche oder das eingeschlossene Volumen verringert bzw. vergrößert werden können. Die federmechanische Verriegelung kann durch Einstellung der Vorspannung der Feder 46 hinsichtlich der Haltekraft variiert werden.

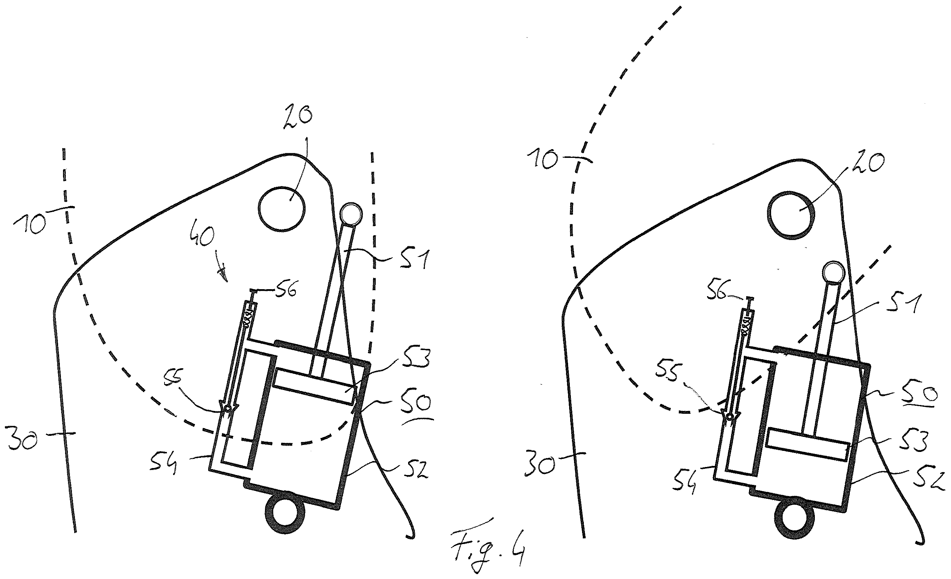

In der Figur 4 ist eine weitere Variante der Erfindung dargestellt. Die Halteeinrichtung 40 ist hydraulisch ausgebildet und bildet somit gleichzeitig eine Dämpfereinrichtung 50 aus. Die Halteeinrichtung 40 mit der Dämpfereinrichtung 50 weist eine Kolbenstange 51 auf, die an dem Oberteil 10 schwenkbar gelagert ist. An dem Ende der Kolbenstange 51 ist ein Kolben 53 angeordnet, der in einem Hydraulikzylinder 52 geführt ist, der wiederum gelenkig an dem Unterteil 30 festgelegt ist. Der Kolben 53 unterteilt den Zylinder 52 in eine Extensionskammer und eine Flexionskammer. Bei einer Flexion wird die Hydraulikflüssigkeit durch einen Bypass 54 von der Flexionskammer in die Extensionskammer geleitet. Innerhalb des Bypasses 54 ist ein Rückschlagventil 55 angeordnet, das über eine Feder vorgespannt ist. Die Federvorspannung ist über eine Einstelleinrichtung 56, beispielsweise eine Schraube, einstellbar. Wird ein Flexionsmoment aufgebracht, das die Federvorspannung des Rückschlagventils 50 übersteigt, öffnet sich das Rückschlagventil 55 und die Hydraulikflüssigkeit kann von der Flexionskammer durch den Bypass 54 in die Extensionskammer und gegebenenfalls einen Ausgleichsbehälter strömen. Das Gelenk, z.B. Knie oder Ellenbogen, beugt sich gegen den entstehenden Strömungswiderstand und lässt eine Flexion zu. Die Flexion wird somit freigegeben, die Flexionsbewegung wird gedämpft. Bei einer Streckung strömt das Hydraulikfluid aus der Extensionskammer durch einen nicht darstellten Rückströmkanal, der ebenfalls mit einem allerdings umgekehrt wirksamen Rückschlagventil versehen ist, zurück in die Flexionskammer. Die gebeugte Stellung des Gelenkes ist in der rechten Darstellung gezeigt.

Der Strömungswiderstand ist durch die Federvorspannung und die Einstellvorrichtung 56 einstellbar, auch ist es möglich eine Umschaltung zwischen zwei Vorspannungen und damit auch Auslösemomenten vorzunehmen oder auch unterschiedliche Widerstände im entriegelten und damit einer Flexion zugänglichen Stellung zu erreichen.

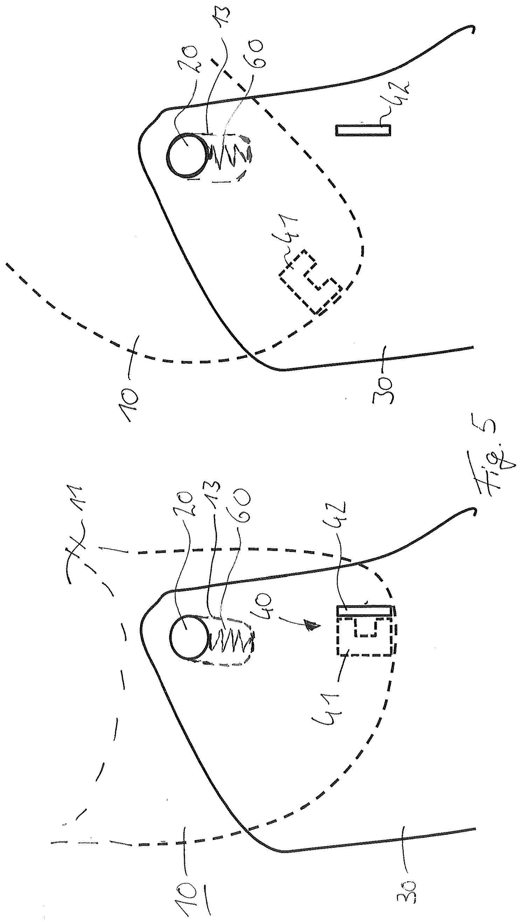

Eine weitere Variante der Erfindung ist in der Figur 5 dargestellt, bei der exemplarisch anhand einer magnetischen Verriegelung eine Verlagerbarkeit des Oberteils 10 relativ zu dem Unterteil 30 entgegen einer Federkraft dargestellt ist. Im dargestellten Ausführungsbeispiel ist das Oberteil 20 mit einer Achse um die Schwenkachse 20 in einem Langloch 13 geführt, das in dem Unterteil 30 ausgebildet ist. Innerhalb des Langloches 13 ist eine Feder 60 angeordnet, die einer Verlagerung entlang des Langloches 13 entgegenwirkt. Wird eine erhöhte Axialkraft entlang des Langloches 13 aufgebracht, verlagert sich der Magnet 41 relativ zu dem Joch 42 und verringert die Haltekraft zwischen dem Magneten 41 und dem Joch 42 und somit auch das aufzubringende Beugemoment, um die Flexion zu ermöglichen. Entsprechende Ausgestaltungen sind auch für die Varianten der Figuren 2 und 3 vorgesehen. Neben einer Verringerung des notwendigen Flexionsmomentes kann durch eine Verlagerung von Oberteil 10 zu dem Unterteil 30 auch eine Erhöhung des aufzubringenden Flexionsmomentes eingestellt werden.

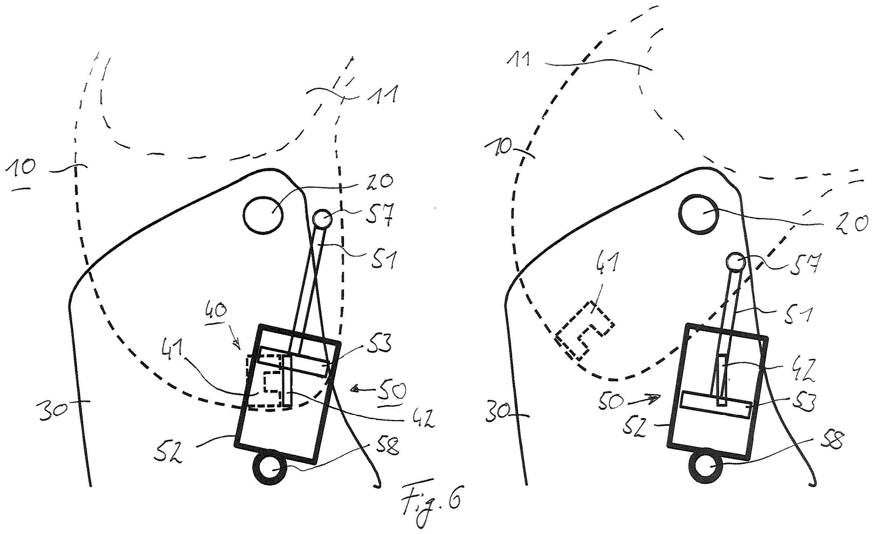

Figur 6 zeigt eine Variante der Figur 1, bei der zusätzlich zu der Halteeinrichtung 40 mit einem Magneten 41 und einem Joch 42 ein Hydraulikdämpfer 50 zwischen dem Oberteil 10 und dem Unterteil 30 angeordnet ist. Der Hydraulikdämpfer 50 ist analog zu der hydraulisch wirksamen Halteeinrichtung 40 gemäß Figur 4 aufgebaut und weist ein Gehäuse 52 auf, in dem ein Zylinder 53 verschieblich gelagert ist. Der Zylinder 53 unterteilt das Gehäuse 52 in zwei Kammern, eine Extensionskammer und eine Flexionskammer, die über Überströmkanäle und gegebenenfalls Ausgleichsbehälter strömungstechnisch miteinander gekoppelt sind. Der hydraulische Widerstand innerhalb der Überströmkanäle ist einstellbar, gegebenenfalls ist Druck in einem Druckspeicher aufbaubar, um eine Umkehrbewegung zu unterstützen.

In dem dargestellten Ausführungsbeispiel ist der Kolben 53 mit einer Kolbenstange 51 an einem oberen Lagerpunkt 57 an dem Oberteil 10 festgelegt. Der obere Lagerpunkt 57 befindet sich posterior der Schwenkachse 20. Der Zylinder 52 ist an dem Unterteil 30 an einem unteren Lagerpunkt 58 angeordnet. Wird das Gelenk aus der extendierten Stellung gemäß der linken Darstellung der Figur 6 eingebeugt, nachdem die magnetisch wirksame Halteeinrichtung 40 überwunden und ein ausreichend großes Beugemoment um die Schwenkachse 20 aufgebracht wurde, verschwenkt das Oberteil 10 relativ zu dem Unterteil 30. Aufgrund der Relativbewegung des oberen Lagerpunktes 57 zu dem unteren Lagerpunkt 58 wird der Hydraulikkolben 53 in Richtung auf den unteren Lagerpunkt 58 bewegt. Hydraulikfluid strömt von der unteren Kammer in die obere Kammer, der hydraulische Widerstand innerhalb des Hydraulikdämpfers 50 dämpft die Beugebewegung des Oberteils 10 relativ zu dem Unterteil 30. Der hydraulische Widerstand und damit auch die Dämpfung der Einbeugung sind einstellbar, beispielsweise durch manuell oder motorisch einstellbare Ventile oder Drosseln innerhalb der Überströmkanäle. Bei einer entgegengesetzten Bewegung, also bei einer Streckbewegung, entfernt sich der obere Lagerungspunkt 57 relativ von dem unteren Lagerungspunkt 58, der Kolben 53 wird in entgegengesetzter Richtung bewegt und eine Extensionsbewegung wird korrespondierend gedämpft. Es können unterschiedlich große Dämpfungswiderstände für die Extensionsbewegung oder Flexionsbewegung vorgesehen sein.

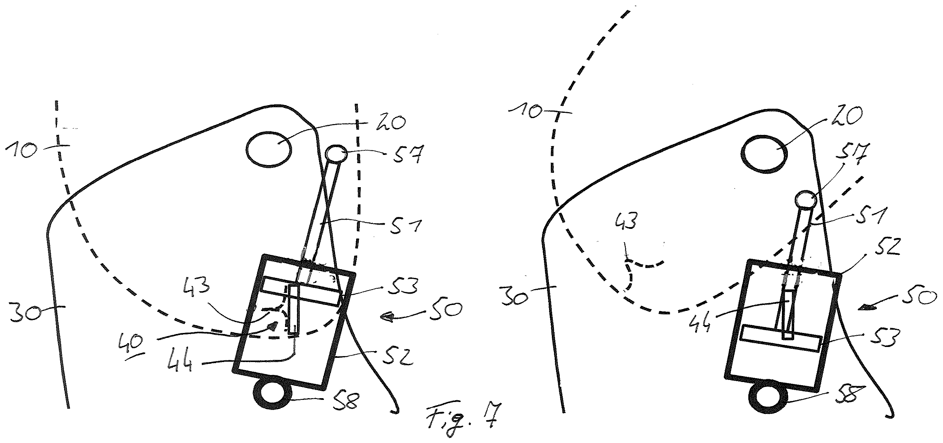

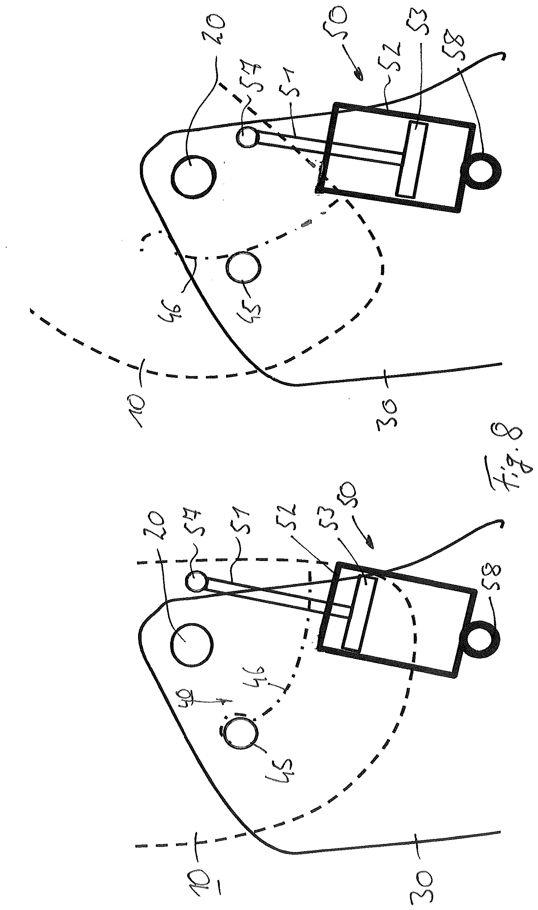

Figur 7 zeigt die Variante der Figur 2 mit der Ausgestaltung der Halteeinrichtung 40 als Saugnapf 43 und einer glatten Fläche 44 in Kombination mit dem Hydraulikdämpfer 50, wie er in der Figur 6 beschrieben wurde. Die Figur 8 zeigt eine Variante der Figur 3 mit der federmechanischen Halteeinrichtung 40 in Verbindung mit einem separaten Hydraulikdämpfer 50 gemäß Figur 6. Die jeweiligen Ausführungen zu den Halteeinrichtungen gemäß der Figuren 2 und 3 gelten entsprechend in Kombination mit den Ausführungen zu dem Hydraulikdämpfer gemäß der Figur 6.

Ein Anwendungsbeispiel der Erfindung betrifft Orthesen mit einer oben beschriebenen orthopädietechnischen Gelenkeinrichtung, die eine Halteeinrichtung aufweisen, die bei Überschreiten einer aufgebrachten Kraft den Widerstand gegen eine Flexion aufhebt oder stark verringert, um z.B. bei einer Spastik Verletzungen zu vermeiden. Dabei werden federbelastete oder gedämpfte orthopädietechnische Gelenkeinrichtungen eingesetzt, sogenannte dynamische Gelenke, um Kontrakturen entgegenzuwirken oder durch Dehnübungen zu therapieren. Hier wirkt die Halteeinrichtung als Überlastschutz.

## Patentansprüche

1. Orthopädietechnische Gelenkeinrichtung mit einem Oberteil (10), an dem obere Anschlussmittel (11) zur Festlegung an einem Patienten angeordnet sind, und einem gelenkig um eine Schwenkachse (20) an dem Oberteil (10) befestigten Unterteil (30), wobei zwischen dem Oberteil (10) und dem Unterteil (30) eine Halteeinrichtung (40) angeordnet ist, wobei die Halteeinrichtung (40) flexionsmomentgesteuert ausgebildet ist und eine Flexion sperrt und bei Überschreiten eines vorbestimmten Flexionsmomentes um die Schwenkachse (20) die Flexion freigibt, **dadurch gekennzeichnet, dass** die orthopädietechnische Gelenkeinrichtung als Orthesen- oder Prothesenkniegelenk oder Orthesen- oder Prothesenellenbogengelenk oder als Orthesen- oder Prothesenhandgelenk ausgebildet ist und die Halteeinrichtung (40) zumindest eine Magnetkopplung (41, 42) und/oder eine Saugnapfkopplung (43, 44) und/oder eine vorgespannte Schnappfeder (45, 46) und/oder eine hydraulische Sperreinheit mit einem schaltbaren Sperrventil (55) aufweist.

2. Orthopädietechnische Gelenkeinrichtung nach Anspruch 1, **dadurch gekennzeichnet, dass** zwischen dem Oberteil (10) und dem Unterteil (30) eine Dämpfereinrichtung (50) angeordnet ist, die eine Flexionsbewegung und/oder Extensionsbewegung des Unterteils (30) relativ zu dem Oberteil (10) dämpft.

3. Orthopädietechnische Gelenkeinrichtung nach Anspruch 2, **dadurch gekennzeichnet, dass** die Dämpfereinrichtung (50) zwischen einem hohen und einem geringen Dämpfungswiderstand schaltbar ausgebildet ist.

4. Orthopädietechnische Gelenkeinrichtung nach Anspruch 3, **dadurch gekennzeichnet, dass** der Dämpfereinrichtung (50) eine axialkraftabhängige Schalteinrichtung zugeordnet ist, die bei Unterschreiten einer auf das Unterteil (30) einwirkenden Axialkraft den verringerten Dämpfungswiderstand freischaltet.

5. Orthopädietechnische Gelenkeinrichtung nach einem der Ansprüche 3 oder 4, **dadurch gekennzeichnet, dass** der Dämpfereinrichtung (50) ein mechanischer Schalter oder ein sensorgesteuerter, mit einem Aktuator versehener Schalter zugeordnet ist.

6. Orthopädietechnische Gelenkeinrichtung nach einem der voranstehenden Ansprüche, **dadurch gekennzeichnet, dass** die Halteeinrichtung (40) als kraftschlüssig oder formschlüssig wirkende Halteeinrichtung (40) ausgebildet ist.

7. Orthopädietechnische Gelenkeinrichtung nach einem der voranstehenden Ansprüche, **dadurch gekennzeichnet, dass** die durch die Halteeinrichtung (40) ausgeübte Haltekraft einstellbar ist.

8. Orthopädietechnische Gelenkeinrichtung nach einem der voranstehenden Ansprüche, **dadurch gekennzeichnet, dass** das Oberteil (10) gegenüber dem Unterteil (30) verschieblich gelagert ist.

9. Orthopädietechnische Gelenkeinrichtung nach Anspruch 8, **dadurch gekennzeichnet, dass** zwischen dem Oberteil (10) und dem Unterteil (30) ein Federelement angeordnet ist, dass einer Verschiebung entgegenwirkt.

10. Orthopädietechnische Gelenkeinrichtung nach einem der voranstehenden Ansprüche, **dadurch gekennzeichnet, dass** zusätzlich zu der Halteeinrichtung (40) ein separater Hydraulikdämpfer (50) zwischen dem Oberteil (10) und dem Unterteil (30) angeordnet ist.

11. Orthopädietechnische Gelenkeinrichtung nach einem der voranstehenden Ansprüche, **dadurch gekennzeichnet, dass** die Halteeinrichtung (40) einen sensorlos arbeitenden Freigabemechanismus (41, 42; 43, 44; 45, 46) aufweist oder ausbildet.

## Claims

1. An orthopedic joint device having an upper part (10), on which are arranged upper connection means (11) for securing to a patient, and having a lower part (30), which is fastened on the upper part (10) in an articulated manner about a pivot axis (20), wherein a retaining device (40) is arranged between the upper part (10) and the lower part (30), wherein the retaining device (40) is of flexion-moment-controlled design and arrests flexion and, when a predetermined flexion moment about the pivot axis (20) is exceeded, releases the flexion, **characterized in that** the orthopedic joint device is designed in the form of an orthotic or prosthetic knee joint or orthotic or prosthetic elbow joint or in the form of ankle joint or wrist joint and that the retaining device (40) has at least one magnetic coupling (41, 42) and/or a suction-cup coupling (43, 44) and/or a prestressed snap spring (45, 46) and/or a hydraulic arresting unit with a switchable arresting valve (55).

2. The orthopedic joint device as claimed in claim 1, **characterized in that** a damper device (50) is arranged between the upper part (10) and the lower part (30) and damps a flexion movement and/or extension movement of the lower part (30) relative to the upper part (10).

3. The orthopedic joint device as claimed in claim 2, **characterized in that** the damper device (50) is designed such that it can be switched between a high damping resistance and a low damping resistance.

4. The orthopedic joint device as claimed in claim 3, **characterized in that** the damper device (50) is assigned an axial-force-dependent switching device which, when the axial force acting on the lower part (30) drops below a certain value, enables the reduced damping resistance.

5. The orthopedic joint device as claimed in either of claims 3 and 4, **characterized in that** the damper device (50) is assigned a mechanical switch or a sensor-controlled switch provided with an actuator.

6. The orthopedic joint device as claimed in one of the preceding claims, **characterized in that** the retaining device (40) is designed in the form of a force-fitting or form-fitting retaining device (40).

7. The orthopedic joint device as claimed in one of the preceding claims, **characterized in that** it is possible to adjust the retaining force exerted by the retaining device (40).

8. The orthopedic joint device as claimed in one of the preceding claims, **characterized in that** the upper part (10) is mounted in a displaceable manner in relation to the lower part (30).

9. The orthopedic joint device as claimed in claim 8, **characterized in that** a displacement-counteracting spring element is arranged between the upper part (10) and the lower part (30).

10. The orthopedic joint device as claimed in one of the preceding claims, **characterized in that,** in addition to the retaining device (40), a separate hydraulic damper (50) is arranged between the upper part (10) and the lower part (30).

11. The orthopedic joint device as claimed in one of the preceding claims, **characterized in that** the retaining device (40) has, or forms, a sensorless-operation release mechanism (41, 42; 43, 44; 45, 46).

## Revendications

1. Dispositif d'articulation orthopédique comprenant une partie supérieure (10) sur laquelle sont disposés des moyens de raccordement supérieurs (11) destinés à être immobilisés au niveau d'un patient, et une partie inférieure (30) fixée à la partie supérieure (10) de manière articulée autour d'un axe de pivotement (20), un dispositif de retenue (40) étant disposé entre la partie supérieure (10) et la partie inférieure (30),
dans lequel le dispositif de retenue (40) est conçu de manière à être commandé par le couple de flexion et bloque une flexion et libère la flexion lorsqu'un couple de flexion prédéterminé autour de l'axe de pivotement (20) est dépassé vers le haut,
**caractérisé en ce que** le dispositif d'articulation orthopédique est conçu comme une articulation orthétique ou prothétique du genou ou comme une articulation orthétique ou prothétique du coude ou comme une articulation orthétique ou prothétique de la main, et
le dispositif de retenue (40) présente au moins un couplage magnétique (41, 42) et/ou un couplage à ventouse (43, 44) et/ou un ressort d'encliquetage précontraint (45, 46) et/ou une unité de blocage hydraulique comprenant une valve de blocage commutable (55).

2. Dispositif d'articulation orthopédique selon la revendication 1, **caractérisé en ce qu'**un dispositif d'amortissement (50) est disposé entre la partie supérieure (10) et la partie inférieure (30), lequel amortit un mouvement de flexion et/ou un mouvement d'extension de la partie inférieure (30) par rapport à la partie supérieure (10).

3. Dispositif d'articulation orthopédique selon la revendication 2, **caractérisé en ce que** le dispositif d'amortissement (50) est conçu de manière à pouvoir être commuté entre une résistance d'amortissement élevée et une résistance d'amortissement faible.

4. Dispositif d'articulation orthopédique selon la revendication 3, **caractérisé en ce qu'**un dispositif de commutation dépendant de la force axiale est associé au dispositif d'amortissement (50), lequel applique la résistance d'amortissement réduite lorsqu'une force axiale agissant sur la partie inférieure (30) est dépassée vers le bas.

5. Dispositif d'articulation orthopédique selon l'une des revendications 3 ou 4,
**caractérisé en ce qu'**un interrupteur mécanique ou un interrupteur commandé par un capteur et pourvu d'un actionneur est associé au dispositif d'amortissement (50).

6. Dispositif d'articulation orthopédique selon l'une des revendications précédentes,
**caractérisé en ce que** le dispositif de retenue (40) est conçu comme un dispositif de retenue (40) agissant par coopération de force ou par coopération de forme.

7. Dispositif d'articulation orthopédique selon l'une des revendications précédentes,
**caractérisé en ce que** la force de retenue exercée par le dispositif de retenue (40) est réglable.

8. Dispositif d'articulation orthopédique selon l'une des revendications précédentes,
**caractérisé en ce que** la partie supérieure (10) est montée de manière mobile en translation par rapport à la partie inférieure (30).

9. Dispositif d'articulation orthopédique selon la revendication 8, **caractérisé en ce qu'**un élément à ressort est disposé entre la partie supérieure (10) et la partie inférieure (30), lequel s'oppose à une translation.

10. Dispositif d'articulation orthopédique selon l'une des revendications précédentes,
**caractérisé en ce que**, en supplément au dispositif de retenue (40), un amortisseur hydraulique (50) séparé est disposé entre la partie supérieure (10) et la partie inférieure (30).

11. Dispositif d'articulation orthopédique selon l'une des revendications précédentes,
**caractérisé en ce que** le dispositif de retenue (40) comprend ou constitue un mécanisme de libération (41, 42 ; 43, 44 ; 45, 46) qui fonctionne sans capteur.
